# EUROPEAN PATENT SPECIFICATION

(11) **EP 0 964 834 B1**
(45) Date of publication and mention of the grant of the patent: **23.07.2003**
(21) Application number: 98907068.5
(22) Date of filing: 26.02.1998
(51) Int. Cl.: C03C 21/00, A61K 6/06, C03C 12/00

(54) **INTRODUCING FLUORIDE IONS INTO GLASS PARTICLES**
EINFÜHRUNG VON FLUORID-IONEN IN GLASTEILCHEN
INTRODUCTION D'IONS FLUORURE DANS DES PARTICULES DE VERRE

(30) Priority: 03.03.1997 GB 9704104
(43) Date of publication of application: 22.12.1999
(73) Proprietor: S&C Polymer GmbH, 25335 Elmshorn (DE)
(72) Inventor: AKINMADE, Ademola, Olaseni, Calne, Wiltshire SN11 8LS (GB)
(74) Representative: James, Michael John Gwynne
(86) International application number: GB9800607
(87) International publication number: WO98039263

(56) References cited:
- DE-A- 1 904 660
- GB-A- 1 193 306
- GB-A- 2 077 281
- GB-A- 2 180 833
- GB-A- 2 202 221
- Kirk-Othmer, Encyclopedia of Chemical Technology, 1994, Fourth Ed., Vol. 11, pp 287-289

## Description

This invention relates to a method of introducing fluoride ions into glass particles. Glass particles so treated may have a variety of uses.

For example, such glass particles may be used in making a particulate material by reacting them(if acid-degradable) to completion with an acid polymer, by an acid-base cement-forming reaction, and grinding the product. "Glass" in this specification can include, according to the context, gelatinising minerals such as bentonite or other mixed metal oxides.

The resulting particulate material can be considered as a glass-depleted cement gel, and can be used in a number of distinct light cured dental materials, such as a liner, fissure sealant, bonding agent/adhesive and anterior restorative. It may also be incorporated in existing cement formulations as a fluoride releasing agent, e.g. incorporation in non-resin based cements and also existing resin based cements.

In conventional glass ionomer cements, aluminosilicate glass is reacted with acid. The outer layer of each glass particle is depleted of metal ions and is degraded to a silica gel. The metal ions have been released by the action of the acid to migrate to the surrounding liquid phase, where they are initially soluble but then accumulate to cause gelation and become insoluble. The resulting cement thus has the form of dispersed particles each having an unreacted aluminosilicate glass core surrounded by a metal-depleted silica gel shell, the shell being about ½ micron thick, the particles being dispersed in a gelated matrix of polyacid chains to which the metal ions which were released from the glass are bound. The shell attained this thickness of about ½ micron during the period while the material surrounding it remained liquid and reactive. The shell ceases to thicken when the surrounding material has set.

If using submicron particles, the unreacted aluminosilicate glass cores would no longer remain. As the glass particles prior to reaction do not exceed 2 microns, preferably not exceeding 1½ microns and ideally not exceeding 1 micron, and are preferably spherical, the acid can release all the metal ions throughout the full depth of each particle before the system gels, leaving simply silica gel cores dispersed in a gelated matrix of polyacid chains to which the metal ions which were released from the glass are bound. The more reactive the acid, the larger the size of glass particle that can be wholly reacted, 1.2 - 1.4 µm being conceivable. Such a hydrogel material is levigated for further use. Note that, all the glass having been leached by acid, no unwanted fresh unreacted glass surface can be exposed by such levigation or particularisation, and the hydrogel material is therefore inert in the presence of water and/or cement-forming acids.

An alternative use for glass treated according to the present invention is (when present in stoichiometric excess) as a component of a glass ionomer cement.

According to the present invention, a method of introducing fluoride ions into glass particles not exceeding 2 micron, comprises contacting the glass with a solution of an ammonium fluoride. Preferably the solvent is added to the glass and fluoride.

Preferably, the fluoride solution is one from which ammonia can evolve, such as ammonium hydrogen difluoride NH₄.HF₂, ammonium fluoride, ammonium heptafluorotantalate (V), ammonium hexafluorogermanate (IV), ammonium hexafluoroniobate, ammonium hexafluorophosphate, ammonium hexafluorosilicate, ammonium hexafluorotitanate (IV), ammonium tetrafluoroborate, ammonium trifluoroacetate or ammonium trifluoromethanesulfonate. Low melting point (for ammonium hydrogen difluoride it is 125C) and high acidity of the aqueous solution of these chemicals enhance their fluoride imparting action, and ammonium hydrogen difluoride is preferred. Fluoride will substitute into the glass irrespective of particle size, but glasses not exceeding 1½ microns, optionally not exceeding 1 micron, are preferred, increasing the ease with which fluoride ions are introduced throughout the thickness of the glass, and the glass particles are preferably spherical. Having said this, the F-containing glass made according to the invention and resulting cement could usefully be used as fluoride reservoir in carboxylate systems, even if less effective than sub-micron glasses. Bentonite, for example, would not be reactive enough to participate in an acid-base reaction, but its reactivity can be improved by introducing fluoride as set forth above, typically by reacting the Al₂O₃ to form AlF₃. Thus, the glass may be an aluminosilicate or otherwise acid-degradable.

The use of ammonium salts is preferable to employing HF, to avoid risks. The use of ammonium cations and fluoriferous anions will deposit F and no cations. Deposited cations will change the composition of the glasses, a complication avoided by the present invention.

The use of ammonium hydrogen difluoride or its analogues deactivates the surfaces of glasses by the crystallisation of calcium fluoride. In the formation and use of glass ionomer cements (glass polyalkenoate cements) from glass particles fluorinated according to the invention, increases in working time with the addition of increased quantities of ammonium hydrogen difluoride are observed.

The use of this ammonium salt on fluoride-free glass can even result in fluoride-containing non-setting glasses, as well as snap setting glasses. The former enables glass powders to be reacted with poly(acrylic acid) and tartaric acid in the presence of water to achieve neutralisation before gelation. Achieving neutralisation before gelation is a prerequisite to the formation of glass core-free cements. As to the latter, the snap-setting glasses, resulting in cements that can set rapidly at the ends of their working times, within 20 seconds, may be used in glass-ionomer cements, which could rival command cure resin-based cements in their rapidity of set and low oral solubility.

This method may be used on glass to be made into a particulate material as set forth above, whereby such material becomes suitable for use in a dental cement formulation as a fluoride release reservoir, and can be considered as a "reactive filler" compatible with most if not all dental material curing routes.

The invention makes possible single-paste resin systems, such as a light-curable paste comprising a cross-linkable monomer, a light-activated initiator and a particulate material as set forth above. Such paste may further comprise a zinc-containing glass, a polyalkenoic acid and optionally water so packed as to keep the pH below 4, and optionally contains Ba, Sr or other divalent glass-forming radio-opaque cation from the reacted glass. Another paste may further comprise an aqueous solution of an acid polymer capable of participating in a glass ionomer setting reaction. These two pastes may be presented in a two-paste cement pack, packed out of contact with each other (and preferably in opaque and/or air-tight packaging as appropriate) until the time of use. In such a pack, preferably one or both pastes further comprise a non-zinc acid-degradable glass and/or a polymeric acid and/or water, such that neither paste reacts until it is unpacked and/or mixed with the other paste.

Optionally, the glass is of particles of radius larger than the depth to which fluoride ions have been introduced when, with polyalkenoic acid and water and preferably without chelating agent, it can form a glass ionomer cement. Preferably in this option, the glass to be fluoridated is added to the fluoride solution.

The invention will now be described by way of example.
- Examples 1-3: describe a method of fluoridating a glass according to the invention. and
- Example 4: describes the use of the resulting glass to make a particulate material being a glass-depleted cement gel,
- Example 5: describes another method according to the invention, and.
- Example 6: describes the use of the glass resulting from Example 5 to make a glass ionomer cement.

### Example 1

1) 2.877 g of ammonium hydrogen difluoride NH₄.HF₂ was added to 18.0 g of water in a PTFE beaker and dissolved.
2) To this was added 8.634 g of sub-micron glass (spherical particles) of composition 120.0 g silica; 102.0 g of alumina and 112.0 g of calcium oxide.
3) The resulting slurry was stirred vigorously until the (immediate and strong) evolution of ammonia ceased, about 2 hours.
4) The slurry was calcined in an oven over 24 hours via a progressive increase in temperature from 98°C to 250°C. The resulting fluoridated glass was an off-white powder.

### Example 2

Example 1 was repeated, but using 2.158 g of ammonium hydrogen difluoride in step 1) instead of the 2.877 g. At step 4), the slurry was heat-treated for 24 hours at 220°C.

### Example 3

Example 1 was repeated, but using 1.439 g of ammonium hydrogen difluoride in step 1) instead of the 2.877 g.

In Examples 1-3, as submicron glass is used, it (in particular the Ca ions in it) becomes fully fluoridated throughout, even if the order of addition of reagents is changed; thus the NH₄.HF₂ may equally successfully be mixed dry with the glass, with the water stirred in afterwards. This latter order is preferred if the glass exceeds 1 micron and is intended for the use specified in Example 4. With that latter order of addition of reagents, the evolution of ammonia is noticeably steadier and slower.

### Example 4 : The following further steps were performed on the product of each of Examples 1-3:

5) The resulting glass (5 parts) was mixed with 0.4 parts of dry poly(acrylic acid) and 0.15 parts tartaric acid to make 5.55 parts of a powder component.
6) 8.33 parts of a liquid component were made from 4.90 parts of water, 2.45 parts of dry poly(acrylic acid) and 0.98 parts of tartaric acid.
7) The 5.55 parts of the powder component were mixed with the 8.33 parts of the liquid component to degrade or dissolve the glass and form a cement gel. The glass was totally deactivated by the fluoridation and, though gelling in the acid, did not set.
8) The resulting cement gel was dried in an oven at 100-105°C for 24 hours to cure.
9) The cured cement gel was ground and passed through a 45 micron sieve. The sieved lot may be referred to as fluoridated glass-depleted cement, or ground cement, or in other terminology an ionomer gel. It may be used as a fluoride-releasing component in resin-based dental restorative material.

The amount of ammonium hydrogen difluoride used in Example 1 was selected to replace all the oxygen from the calcium oxide of the glass with fluoride ions. The lesser amounts in Examples 2 and 3 were (obviously) meant to achieve less than total replacement. The level of fluoride in the glass would have a bearing on the fluoride release from the resulting resin-modified cement, the reactivity of the glass in cement formation, and the strength of the resulting glass-ionomer cements. In these Examples, no temperature above 220C is required.

### Example 5

Examples 1 to 3 were repeated using the stated order of addition of reagents NH₄.HF₂ then water then glass. However in Example 5 the glass was of 5-micron spheres. There was the same immediate and strong evolution of ammonia as a vigorous surface reaction took place. The result was glass particles with an outer layer uniformly about ½µm thick rich in crystalline CaF₂ and an inner unaffected core.

### Example 6

The samples of Example 5 were mixed in proportions p:1 = 2:1 (i.e. glass in stoichiometric excess) with 50% aqueous solutions of poly(acrylic acid) PAA of MW = 50000, in the absence of tartaric acid or other chelating agent. The mixtures remained workable for about 2½ minutes and then set almost instantly. It is conjectured that this method surprisingly yielded a successful snap-set glass ionomer cement because the dissolution of the outer CaF₂-rich layer by the PAA took about 2 minutes (leading to no reaction) and, when the PAA reached the underlying unaffected glass, which it did nearly simultaneously on all particles thanks to their uniformity of fluoridation, the glass ionomer setting reaction was sudden and rapid.

### Example 7

Using generally the method of the foregoing Examples, two zinc-based glasses (Z45 and Z72) were fluorinated. Z45 contains no fluoride while Z72 is a fluoride-containing glass. Both however yield glass ionomer cements which release no fluoride ions. This Example is to create fluoride-releasing and workable "conventional" cements from the extremely fast-setting Z45 glasses, and to create releasable fluoride ions from the Z72 glasses and, by extension, light-cured cements deriving from them. The compositions of Z45 and Z72 are described later.

The glasses were fluorinated using ammonium hydrogen difluoride thus:-
The glass in powdered form was stirred into an aqueous solution of the ammonium hydrogen difluoride.
Stirring was effected for up to 12 hours while ammonia is evolved.
The resulting slurry was placed in an oven, preset at 220°C, and stirred occasionally while still in slurry form. It was left at this temperature for 12 hours. This temperature ensures the total decomposition of the ammonium salt.
The dried, paler, annealed glass was dispersed by any suitable means-hand, blender, or ball mill.

Six fluorinations of Z45 glass following the protocol outlined above were carried out, denoted Z45/F/1.6.

**Table 1.**

| The Fluorination of Z45 Glass | | | |
|---|---|---|---|
| | Constituents (weight, g) | | |
| Experiment | Z45 glass | Water | Ammonium salt |
| Z45/F/1 | 50.00 | 50.00 | 2.842 |
| Z45/F/2 | 50.00 | 50.00 | 2.842* |
| Z45/F/3 | 50.00 | 50.00 | 2.000 |
| Z45/F/4 | 50.00 | 50.00 | 3.500 |
| Z45/F/5 | 50.00 | 50.00 | 2.300 |
| Z45/F/6 | 50.00 | 50.00 | 2.540 |

| | | | |
|---|---|---|---|
| * Sequence of addition: glass, salt. water. The sequence for the other five was salt, water, glass. | | | |

The quantities of the ammonium salts used in the fluorination were informed by the quantity of calcium ions contained in the Z45 glass. The composition of the Z45 glass is:

| Ingredients of Z45 | Main Components Weight (g) | Constituents Breakdown (g) |
|---|---|---|
| CaO | 9.24 | 10.01 |
| ZaO | 50.00 | 50.00 |
| Bentonite EX048 | 30.50 | - |
| (silica) | | 22.59 |
| (alumina) | | 5.07 |
| (magnesia) | | 1.00 |
| (potash) | | 0.41 |
| (soda) | | 0.66 |
| | 89.74 | 89.74 |

89.74g of Z45 glass contain 0.1788 moles of Ca and O in the CaO. Substituting F for O, 0.1788 moles of O is equivalent to 3.396g of F. 3.396g of F is contained in 5.098g of ammonium hydrogen difluoride. This is equivalent to 2.842g of the ammonium salt and 50.00g of Z45 glass, the composition of Z45/F/1 and Z45/F/2. Z45/F/3-6 contain less than the amount of F necessary for the stoichiometric conversion of all the O of CaO to F. On the other hand. Z45/F/4 contains a large excess of F ions.

The stoichiometric conversion of all the O of CaO of glass Z72 into F was attempted using ammonium hydrogen difluoride. The composition of Z72 glass is:

| Ingredients of Z72 | Constituents (weight, g) |
|---|---|
| silica | 20.10 |
| alumina | 5.30 |
| lime | 16.10 |
| magnesia | 1.54 |
| soda | 1.02 |
| zinc oxide | 47.50 |
| fluoride | 12.20 |

100g of Z272 contains 16.10g (0.2875 moles) of lime, CaO. This is equivalent of 5.4625 g of F, and 8.1995 g of ammonium hydrogen difluoride. Therefore, 50.00g of Z72 requires 4.10g of the ammonium salt for the stoichiometric conversion of all its CaO into fluoride.

### Properties of Z45/F/x Cements

The Z45/F/x glasses were blended with dry poly(acrylic acid), PAA, and tartaric acid, TA, to form anhydrous cement powders. These were mixed with aqueous poly(acrylic acid) -based solution at powder:liquid ratios (p:1) of 3.6:1 to form cements. The resulting cements are equivalent to mixing 2 parts of Z45/F/x with 1 part of aqueous solution of 50 wt% PAA; and 10wt% TA.

| Constituents of Cement | Weight of Powder | Constituents (g) Liquid |
|---|---|---|
| Z45/F/X | 6.25 | - |
| PAA | 1.062 | 2.00 |
| TA | 0.062 | 1.00 |
| Water | - | 5.00 |

### Handling Properties of Z45/F/x Cement

The working and setting times of the Z45/F/X cements, evaluated at ambient conditions (22°C and 50% RH), were

| Cement made from: | Ammonium Salt Qty Used (g/50g) | Working Time (min) | Setting Time (mins) |
|---|---|---|---|
| Z45 | nil | < 1 | < 1.5 |
| Z45/F/3 | 2.00 | 1.5 | 2.0 |
| Z45/F/5 | 2.30 | 1.7 | 2.5 |
| Z45/F/6 | 2.54 | 1.9 | 2.8 |
| Z45/F/1&2 | 2.84 | 2.3 | 3.3 |
| Z45/F/4 | 3.50 | 2.5 | 4.0 |

Ammonium hydrogen difluoride is seen to increase the working and setting times of cements and create practical cements from the fast-setting Z45 glass. The progressive incorporation of F into the glass network is attended by an increase in working time of the resulting cements, probably by the increasing acidifying (decreasing basicity) action of F ions in the glass.

### Strength of Z45/F/x Cements

The biaxial flexural and compressive strengths of Z45/F/x cements were determined after storage for 24 hours, 1, 2 and 4 weeks in distilled water at 37°C to be:-

| Cement Age | Compressive Strength/Mpa (Standard Deviation of 6 measurements) | | | | |
|---|---|---|---|---|---|
| | Z45/F/3 | Z45/F/5 | Z45/F/6 | Z45/F/1 | Z454/F/4 |
| 24 hrs | 133.50 (4.87) | 142.38 (13.08) | 145.62 (7.78) | 148.01 (2.28) | 142.25 (5.52) |
| 1 week | 154.94 (1.79) | 156.55 (9.50) | 157.82 (4.21) | 156.97 (7.50) | 161.52 (5.67) |
| 2 weeks | 155.24 (9.81) | 162.71 (11.25) | 157.64 (6.54) | 158.66 (6.81) | 162.37 (6.87) |
| 1 month | 152.91 (8.31) | 167.59 (9.22) | 157.97 (10.88) | 171.41 (5.14) | 165.53 (5.89) |

| Cement Age | Biaxial Flexural Strength/MPa (Standard Deviation of 6 measurements) | | | | |
|---|---|---|---|---|---|
| 24 firs | 47.26 (1.88) | 44.10 (1.64) | 49.28 (2.79) | 46.38 (5.15) | 49.12 (0.86) |
| 1 week | 44.94 (4.80) | 46.19 (1.67) | 49.74 (1.85) | 45.98 (4.94) | 49.81 (6.98) |
| 2 weeks | 54.82 (8.62) | 49.90 (3.99) | 46.07 (5.23) | 47.09 (6.11) | 46.67 (5.14) |
| 1 month | 47.12 (9.25) | 52.60 (1.77) | 50.75 (8.60) | 48.02 (3.52) | 53.34 (5.69) |
| Note: The strength values and the appended values in parentheses are the mean and standard deviation values of 6 measurements. | | | | | |

Unlike in the case of the compressive strengths, the biaxial flexural strengths of the cements are not significantly different from one another over the storage period. Overall, Z45/F/1, representing the theoretical stoichiometric conversion of CaO to calcium fluoride, seemed best.

### Fluoride Release From Z45/F/x Cements

The release of fluoride from Z45/F/2, Z45/F/3 and Z45/F/4 cements was evaluated for 1mm x 10mm diameter cement discs in 100 mls of distilled water at 37°C.

The Fluoride Content of these Cements was:

| Cement | Theoretical F in Glass (%) | Determined F in Glass (%) |
|---|---|---|
| Z45/F/3 | 2.66 | 2.33 |
| Z45/F/2 | 3.78 | 3.21 |
| Z45/F/4 | 4.66 | 3.78 |
| Notes: The theoretical F in the glass is calculated from the amount of ammonium hydrogen difluoride used to treat 50g of the glasses; | | |

The determined fluoride (F) in the glass has been obtained using x-ray fluorescence;

The ratio of theoretical/determined F shows that the efficacy of the F treatment decreases with increasing F agent used (theoretical F in glass).

The fluoride release was:-

| Cement Age | Fluoride Released From Z45/F/X/n Cements | | |
|---|---|---|---|
| | Z45/F/3 cumulative F (% of F) | Z45/F/2 cumulative F (% of F) | Z45/F/4 cumulative F (% of F) |
| 1 hour | 0.5 | 0.7 | 0.3 |
| 2 hours | 0.8 | 1.4 | 0.6 |
| 3 hours | 1.1 | 2.2 | 0.9 |
| 4 hours | 1.3 | 2.8 | 1.1 |
| 5 hours | 1.4 | 3.4 | 1.3 |
| 6 hours | 1.5 | 3.8 | 1.6 |
| 24 hours | 3.0 | 7.5 | 3.4 |
| 5 days | 5.0 | 14.4 | 5.8 |
| 8 days | 6.4 | 17.6 | 8.1 |
| 14 days | 8.1 | 21.7 | 10.5 |
| 1 month | 8.7 | 27.8 | 15.6 |
| 2 months | 13.7 | 41.5 | 23.4 |
| 3 months | 16.6 | 54.4 | 29.2 |
| 4 months | 19.1 | 66.0 | 34.3 |
| Note that the fluoride released from the cements did not follow the trend of fluoride content of the cement's constituent glass. However, and more importantly, the levels of fluoride release from the cements is of the oder of magnitude exhibited by commercial polyalkenoate cements. Therefore, as with those cements, the cements in this Example may be expected to confer cariostatic action to restored teeth. | | | |

### Properties of Z72 Cements

Glass Z72 (which already contains fluoride, which however does not leach out) was fluorinated as described. The fluorinated glass ("Z72/F") was used to make resin-modified cements being hybrids of dental resin composites and conventional glass-ionomer cements, cured for 4x20s using blue light (flexural specimens), or prepared in 2mm layers with 40s light curing/layer (compressive specimens).

| COMPONENT | FORMULATIONS | | |
|---|---|---|---|
| | LC48 | LC49 | LC50 |
| 1) Trimethylolpropane trimethacrylate | 0.12 | 0.12 | 0.36 |
| 2) Urethane dimethacrylate | 4.68 | 4,68 | 4.68 |
| 3) 1,6 Hexane diol dimethacrylate | 0.54 | 0.54 | 0.54 |
| 4) Bis(2-methacryloyloxyethyl) pyromellitate | 0.15 | 0.15 | 0.15 |
| 5) Camphorquinone | 0.09 | 0.09 | 0.09 |
| 6) 4-benzoyl-4-methyldiphenyl sulphide | 0.045 | 0.045 | 0.045 |
| 7) Ethyl p-(dimethylamino benzoate) | 0.30 | 0.30 | 0.30 |
| 8) Benzophenone | 0.03 | 0.03 | 0.03 |
| 9) Ethyl methacrylate phosphate | 0.33 | 0.33 | 0.60 |
| 10) 2-(Dimethylamino) ethyl methacrylate | 0.10 | 0.10 | 0.10 |
| 11) Anhydrous Z72/F glass ionomer | 3.00 | 4.00 | 4.00 |
| 12) Barium aluminosilicate | 12.00 | 12.00 | 12.00 |
| 13) Acetone | 0.20 | 0.20 | 0.20 |
| 14) Gamma-Methacryloxypropyltrimethoxysilane | - | 0.10 | 0.16 |
| BIAXIAL FLEXURAL STRENTH - Instantaneous (MPa) | 54.9 | 68.0 | 86.28 |
| - 24 hours (MPa) | 91.2 | 104.2 | - |
| - 1 week (MPa) | 69.5 | 96.7 | - |
| - 1 month (MPa) | 91.7 | - | 82.7 |
| COMPRESSIVE STRENTH - Instantaneous (MPa) | 93.6 | 123.4 | 121.3 |
| - 24 hours (MPa) | 133.8 | 161.6 | - |
| - 1 week (MPa) | 145.9 | 159.1 | - |

The compressive and biaxial flexural strengths of the LC48-50 cements, and those prepared thereafter, were not inferior to the Z72 (i.e. non-ammonium F salt treated) resin comments.

| Cement Age | F Release (micro gram/mm sq) | |
|---|---|---|
| | Z72/F Cements | |
| | F release | Cumulative F |
| 1 hour | 0.152 | 0.152 |
| 2 hours | 0.130 | 0.282 |
| 3 hours | 0.108 | 0.390 |
| 4 hours | 0.132 | 0.522 |
| 5 hours | 0.122 | 0.644 |
| 6 hours | 0.125 | 0.769 |
| 24 hours | 0.137 | 0.906 |
| 5 days | 0.166 | 1.072 |
| 2 weeks | 0.155 | 1.277 |

## Claims

1. A method of introducing fluoride ions into glass particles not exceeding 2 microns, comprising contacting the glass with a solution of an ammonium fluoride.

2. A method according to Claim 1, wherein the ammonium fluoride is ammonium hydrogen difluoride NH₄.HF₂, ammonium fluoride, ammonium heptafluorotantalate (V), ammonium hexafluorogermanate (IV), ammonium hexafluoroniobate, ammonium hexafluorophosphate, ammonium hexafluorosilicate, ammonium hexafluorotitanate (IV), ammonium tetrafluoroborate, ammonium trifluoroacetate or ammonium trifluoromethanesulfonate.

3. A method according to Claim 1 or Claim 2, wherein the glass is an acid-degradable glass.

4. A method according to any preceding claim, wherein the glass is an aluminosilicate glass.

5. A method according to any preceding claim, wherein the particles do not exceed 1½ microns.

6. A method according to Claim 5, wherein the particles do not exceed 1 micron.

7. A method according to any preceding claim, wherein the solvent is added to the glass particles and fluoride.

8. A method according to any of Claims 1 to 6, wherein the glass particles are added to the fluoride solution.

9. A method according to any preceding claim, wherein fluoride ions have been introduced throughout the thickness of the glass particles.

10. Glass particles which have had fluoride ions introduced into them by a method according to any preceding claim.

11. A method according to Claim 3 or 4, wherein the glass is of particles of radius larger than the depth to which fluoride ions have been introduced.

12. A method according to Claim 11, wherein the glass particles are added to the fluoride solution.

13. Glass particles which have had fluoride ions introduced into them by a method according to Claim 11 or 12.

14. A glass ionomer cement comprising a glass according to Claim 13, a polyalkenoic acid or precursor thereof, and water.

15. A glass ionomer cement according to Claim 14, without chelating agent.

## Patentansprüche

1. Verfahren zum Einführen von Fluoridionen in Glaspartikel, welche eine Größe von 2 um nicht übersteigen,
**gekennzeichnet durch,**
Kontaktieren des Glases mit einer Lösung aus einer Ammoniumfluorid-Lösung.

2. Verfahren nach Anspruch 1, **dadurch gekennzeichnet, daß** das Ammoniumfluorid Ammoniumwasserstoffdifluorid NH₄.HF₂, Ammoniumfluorid, Ammoniumheptafluortantalat (V), Ammoniumhexafluorgermanat (IV), Ammoniumhexafluomiobat, Ammoniumhexafluorphosphat, Ammoniumhexafluorsilikat, Ammoniumhexafluortitanat (IV), Ammoniumtetrafluorborat, Ammoniumtrifluoracetat oder Ammoniumtrifluormethansulfonat ist.

3. Verfahren nach Anspruch 2, **dadurch gekennzeichnet, daß** das Glas ein säureabbaubares Glas ist.

4. Verfahren nach einem der vorhergehenden Ansprüche, **dadurch gekennzeichnet, daß** das Glas ein Aluminosilikatglas ist.

5. Verfahren nach einem der vorhergehenden Ansprüche, **dadurch gekennzeichnet, daß** die Partikel eine Größe von 1½ µm nicht übersteigen.

6. Verfahren nach Anspruch 5, **dadurch gekennzeichnet, daß** die Partikel eine Größe von 1 µm nicht übersteigen.

7. Verfahren nach einem der vorhergehenden Ansprüche, **dadurch gekennzeichnet, daß** das Lösungsmittel den Glaspartikeln und dem Fluorid zugegeben wird.

8. Verfahren nach einem der Ansprüche 1 bis 6, **dadurch gekennzeichnet, daß** die Glaspartikel der Fluoridlösung zugegeben werden.

9. Verfahren nach einem der vorhergehenden Ansprüche, **dadurch gekennzeichnet, daß** Fluoridionen über die gesamte Dicke der Glaspartikel eingeführt wurden.

10. Glaspartikel, in die Fluoridionen mittels einem Verfahren gemäß einem der vorhergehenden Ansprüche zugeführt wurden.

11. Verfahren nach Anspruch 3 oder 4, **dadurch gekennzeichnet, daß** das Glas Partikel mit einem Radius umfaßt, welcher größer ist als die Tiefe, in die Fluoridionen eingeführt wurden.

12. Verfahren nach Anspruch 11, **dadurch gekennzeichnet, daß** die Glaspartikel der Fluoridlösung zugegeben werden.

13. Glaspartikel, in die Fluoridionen mittels einem Verfahren gemäß Anspruch 11 oder 12 zugeführt wurden.

14. Glasionomerzement umfassend ein Glas gemäß Anspruch 13, eine Polyalkenoidsäure oder einen Vorläufer dieser und Wasser.

15. Glasionomerzement nach Anspruch 14 ohne Chelatbildungsmittel.

## Revendications

1. Procédé d'introduction d'ions fluorure dans des particules de verre ne dépassant pas 2 microns, comprenant la mise en contact du verre avec une solution d'un fluorure d'ammonium.

2. Procédé selon la revendication 1, dans lequel le fluorure d'ammonium est l'hydrogénodifluorure d'ammonium NH₄.HF₂, le fluorure d'ammonium, l'heptafluorotantalate (V) d'ammonium, l'hexafluorogermanate (IV) d'ammonium, l'hexafluoroniobate d'ammonium, l'hexafluorophospate d'ammonium, l'hexafluorosilicate d'ammonium, l'hexafluorotitanate d'ammonium (IV), le tétrafluoroborate d'ammonium, le trifluoroacétate d'ammonium ou le trifluorométhanesulfonate d'ammonium.

3. Procédé selon la revendication 1 ou la revendication 2, dans lequel le verre est un verre dégradable par les acides.

4. Procédé selon l'une quelconque des revendications précédentes, dans lequel le verre est un verre d'aluminosilicate.

5. Procédé selon l'une quelconque des revendications précédentes, dans lequel les particules ne dépassent pas 1 ½ micron.

6. Procédé selon la revendication 5, dans lequel les particules ne dépassent pas 1 micron.

7. Procédé selon l'une quelconque des revendications précédentes, dans lequel le solvant est ajouté aux particules de verre et au fluorure.

8. Procédé selon l'une quelconque des revendications 1 à 6, dans lequel les particules de verre sont ajoutées à la solution de fluorure.

9. Procédé selon l'une quelconque des revendications précédentes, dans lequel les ions fluorure ont été introduits à travers l'épaisseur des particules de verre.

10. Particules de verre qui ont eu des ions fluorure introduits dans celles-ci par un procédé tel que défini à l'une quelconque des revendications précédentes.

11. Procédé selon l'une des revendications 3 ou 4, dans lequel le verre est de particules de rayon supérieur à la profondeur à laquelle les ions fluorure ont été introduits.

12. Procédé selon la revendication 11, dans lequel les particules de verre sont ajoutées à la solution de fluorure.

13. Particules de verre qui ont eu des ions fluorure introduits dans celles-ci par un procédé tel que défini à l'une des revendications 11 ou 12.

14. Ciment verre ionomère comprenant un verre tel que défini à la revendication 13, un acide polyalcénoïque ou un précurseur de celui-ci, et de l'eau.

15. Ciment verre ionomère selon la revendication 14, sans agent chélatant.
